# EUROPEAN PATENT APPLICATION

(11) **EP 4 527 401 A1**
(43) Date of publication of application: **26.03.2025**
(21) Application number: 23903536.3
(22) Date of filing: 13.12.2023
(51) Int. Cl.: A61K 36/254, A23L 33/105, A61P 1/04, A61P 1/14

(54) **METHOD FOR PRODUCING HOT WATER EXTRACT OF ABOVEGROUND PORTION OF PLANT OF GENUS ARALIA, COMPOSITION FOR PROMOTING EXPRESSION OF UROGUANYLIN OR GUANYLIN, COMPOSITION FOR PROMOTING INTESTINAL PERISTALSIS, COMPOSION FOR SUPPRESSING INTESTINAL INFLAMMATION, AND COMPOSITION FOR IMPROVING INTESTINAL BARRIER FUNCTION**

(30) Priority: 14.12.2022 JP 2022199475
(71) Applicant: Amino Up Co., Ltd., Sapporo-shi Hokkaido 004-0839 (JP)
(72) Inventor: TAKANO Sho, Sapporo-shi, Hokkaido 004-0839 (JP)
(74) Representative: Wasner, Marita
(86) International application number: PCT/JP2023/044639
(87) International publication number: WO 2024/128252

(57) **Abstract**

According to the present invention, a method for producing a hot water extract of an aboveground portion of a plant of the genus aralia is characterized by comprising a step for adding sodium hydrogen carbonate.

## Description

### TECHNICAL FIELD

The present invention relates to a method for producing a hot water extract of an aerial part of a plant of the genus Eleutherococcus, a composition for promoting expression of uroguanylin or guanylin, a composition for promoting intestinal peristalsis, a composition for suppressing intestinal inflammation, and a composition for improving intestinal barrier function.

### BACKGROUND ART

Various studies have revealed that intestinal functions are diverse, and that changes in intestinal functions affect even the general condition.

In recent years, it has been shown that cGMP signals in the intestines are involved in the regulation of the overall intestinal functions, and affect the secretion of intestinal mucus, the formation of intestinal bacterial flora, the formation of tight junctions (barrier function), nutrient absorption, and the like (Non-Patent Literature 1).

As factors involved in the cGMP signal, a guanylate cyclase C receptor (GUCY2C (GC-C)) expressed in the small intestine and the large intestine, uroguanylin (GUCA2B) expressed in the stomach and the intestines (mainly the small intestine), and guanylin (GUCA2A) expressed in the intestines (mainly the large intestine) are known. Both uroguanylin and guanylin are secreted into the intestinal lumens and the blood. The guanylate cyclase C receptor synthesizes cGMP from GTP in cells when accepting uroguanylin or guanylin as a ligand. Therefore, it is considered that uroguanylin and guanylin act as triggers of cGMP signal production, and that the promotion of production thereof improves the intestinal functions.

Root bark of a plant of the family Araliaceae, the genus Eleutherococcus, has been used for alleviating wind-dampness (symptoms of muscles and joints), strengthening muscles and bones, and the like as a Chinese medicine for a long time. In addition, with regard to the effect on the intestines, it has been reported that ukogi tea extracted from leaves of Eleutherococcus sieboldianus activates peristalsis of the intestines (Non-Patent Literature 2). In addition, it has been reported in mice models that an Eleutherococcus senticosus root extract improves contraction of excised ileum specimens and ability of charcoal gastrointestinal transit (Non-Patent Literature 3).

### CITATIONS LIST

### NON-PATENT LITERATURE

Non-Patent Literature 1: Rappaport, J.A., & Waldman, S. A. (2018). Frontiers in Oncology, 2018 Aug 6; 8:299. doi:10.3389/fonc.2018.00299. eCollection 2018. The guanylate cyclase C-cGMP signaling axis opposes intestinal epithelial injury and neoplasia.
Non-Patent Literature 2: Journal of Home Economics of Japan, Vol. 57, No. 7, 497-503 (2006), Effects of Ingesting Ukogi (Acabthpanax sieboldianum) Tea on the Intestinal Environment and Small Intestinal Tissue of Mice
Non-Patent Literature 3: Biological and Pharmaceutical Bulletin VOL. 43, NO. 5, 817-822 (2020), Acanthopanax senticosus Root Extract Exerts Dual Action on Mouse Ileal Smooth Muscle Function, Leading to Modulation of Gastrointestinal Motility

### SUMMARY OF INVENTION

### TECHNICAL PROBLEMS

However, not all active ingredients of the family Araliaceae, the genus Eleutherococcus, that act on the intestines, have been elucidated, and no efficient method for producing an Eleutherococcus extract for effectively improving the functions and conditions of the intestines has been clarified.

In view of the above circumstances, the present inventors have found, as a result of extensive studies, that using the plant of the genus Eleutherococcus, more specifically the aerial part of the plant of the genus Eleutherococcus, a hot water extract of an aerial part of a plant of the genus Eleutherococcus, that is obtained by a production method including a step of adding sodium bicarbonate, has a remarkable effect of promoting expression of uroguanylin or guanylin, and have completed the present invention. An object of the present invention is to provide a method for efficiently producing a hot water extract of an aerial part of a plant of the genus Eleutherococcus excellent in effect of promoting expression of uroguanylin or guanylin, effect of promoting intestinal peristalsis, effect of suppressing intestinal inflammation, and effect of improving intestinal barrier function, a composition for promoting expression of uroguanylin or guanylin, a composition for promoting intestinal peristalsis, a composition for suppressing intestinal inflammation, and a composition for improving intestinal barrier function.

### SOLUTIONS TO PROBLEMS

In order to achieve the above object, a method for producing a hot water extract of an aerial part of a plant of the genus Eleutherococcus according to a first aspect of the present invention includes:
a step of adding sodium bicarbonate.

For example, the hot water extract of the aerial part of the plant of the genus Eleutherococcus promotes expression of uroguanylin or guanylin.

For example, the plant of the genus Eleutherococcus is Eleutherococcus senticosus.

A composition for promoting expression of uroguanylin or guanylin according to a second aspect of the present invention
contains as an active ingredient a hot water extract of an aerial part of a plant of the genus Eleutherococcus.

For example, the hot water extract of the aerial part of the plant of the genus Eleutherococcus is produced by adding sodium bicarbonate.

For example, the plant of the genus Eleutherococcus is Eleutherococcus senticosus.

A composition for promoting intestinal peristalsis according to a third aspect of the present invention
contains as an active ingredient a hot water extract of an aerial part of a plant of the genus Eleutherococcus produced by adding sodium bicarbonate.

For example, the plant of the genus Eleutherococcus is Eleutherococcus senticosus.

A composition for suppressing intestinal inflammation according to a fourth aspect of the present invention
contains as an active ingredient a hot water extract of an aerial part of a plant of the genus Eleutherococcus produced by adding sodium bicarbonate.

For example, the plant of the genus Eleutherococcus is Eleutherococcus senticosus.

A composition for improving intestinal barrier function according to a fifth aspect of the present invention
contains as an active ingredient a hot water extract of an aerial part of a plant of the genus Eleutherococcus produced by adding sodium bicarbonate.

For example, the plant of the genus Eleutherococcus is Eleutherococcus senticosus.

### ADVANTAGEOUS EFFECTS OF INVENTION

The present invention can provide a method for efficiently producing a hot water extract of an aerial part of a plant of the genus Eleutherococcus excellent in effect of promoting expression of uroguanylin or guanylin, effect of promoting intestinal peristalsis, effect of suppressing intestinal inflammation, and effect of improving intestinal barrier function, a composition for promoting expression of uroguanylin or guanylin, a composition for promoting intestinal peristalsis, a composition for suppressing intestinal inflammation, and a composition for improving intestinal barrier function.

### BRIEF DESCRIPTION OF DRAWINGS

Fig. 1 includes graph diagrams showing increases in relative expressions of uroguanylin and guanylin by a hot water extract of Eleutherococcus senticosus in the Examples, wherein Fig. 1(a) is a graph diagram of relative expression levels of uroguanylin, and Fig. 1(b) is a graph diagram of relative expression levels of guanylin.
Fig. 2 is a graph diagram showing a comparison between the relative expression levels of guanylin by the hot water extract of Eleutherococcus senticosus in the Example and relative expression levels of guanylin by Eleutherococcus senticosus extracts produced by adding another alkali source.
Fig. 3 is a graph diagram showing a comparison between the relative expression levels of guanylin by the hot water extract of Eleutherococcus senticosus in the Example and relative expression levels of guanylin by Eleutherococcus senticosus extracts produced by adding other alkali sources.
Fig. 4 is a diagram illustrating methods for producing extracts A to E.
Fig. 5 is a graph diagram showing a comparison among relative expression levels of guanylin by the extracts A to E.
Fig. 6 is a graph diagram showing a comparison between the relative expression levels of guanylin by the hot water extract of Eleutherococcus senticosus in the Examples and relative expression levels of guanylin by extracts derived from other plants.
Fig. 7 is a schematic view showing an administration schedule of an animal test using the hot water extract of Eleutherococcus senticosus in the Examples.
Fig. 8 includes graph diagrams of functionality evaluation by an animal test using the hot water extract of Eleutherococcus senticosus in the Examples, wherein Fig. 8(a) is a graph diagram of relative expression levels of uroguanylin, and Fig. 8(b) is a graph diagram of relative expression levels of guanylin.
Fig. 9 includes graph diagrams of functionality evaluation by an animal test using the hot water extract of Eleutherococcus senticosus in the Examples, wherein Fig. 9(a) is a graph diagram of a cGMP concentration in the ileum, and Fig. 9(b) is a graph diagram of a cGMP concentration in the large intestine.
Fig. 10 includes graph diagrams of functionality evaluation by an animal test using the hot water extract of Eleutherococcus senticosus in the Examples, wherein Fig. 10(a) is a graph diagram of relative expression levels of an inflammation marker calprotectin, and Fig. 10(b) is a graph diagram of amounts of TNF-α derived from intestinal tissue per weight of large intestinal tissue.
Fig. 11 includes graph diagrams of functionality evaluation by an animal test using the hot water extract of Eleutherococcus senticosus in the Examples, wherein Fig. 11(a) is a graph diagram of relative expression levels of Claudin 4, Fig. 11(b) is a graph diagram of relative expression levels of JAM-A, and Fig. 11(c) is a graph diagram of relative expression levels of occludin.
Fig. 12 includes graph diagrams of feces weight evaluation by an animal test using the hot water extract of Eleutherococcus senticosus in the Examples, wherein Fig. 12(a) is a graph diagram of a dry feces weight, and Fig. 12(b) is a photograph diagram of dry feces.

### DESCRIPTION OF EMBODIMENTS

### (1. Method for producing hot water extract of aerial part of plant of the genus Eleutherococcus)

A method for producing a hot water extract of an aerial part of a plant of the genus Eleutherococcus according to the present invention includes a step of adding sodium bicarbonate.

For example, a hot water extract of an aerial part of the plant of the genus Eleutherococcus produced by the method according to the present invention can increase an intracellular cGMP concentration through stimulation of a guanylate cyclase C receptor in intestinal mucosal epithelial cells by promoting expression of uroguanylin or guanylin. This can result in obtainment of an effect of promoting intestinal peristalsis, an effect of suppressing intestinal inflammation, and an effect of improving intestinal barrier function, and improvement in functions and conditions of the intestines.

Examples of the plant of the genus Eleutherococcus used in the production method of the present invention include Eleutherococcus senticosus, Eleutherococcus sieboldianus, Eleutherococcus spinosus, and Eleutherococcus sciadophylloides, belonging to the genus Eleutherococcus (Acanthopanax). For example, Eleutherococcus senticosus can be suitably used. As the plant of the genus Eleutherococcus, a plant cultivated for arbitrary days can be used.

In the production method of the present invention, the portion "aerial part" of the plant of the genus Eleutherococcus is used from the viewpoint of the effect of promoting expression of uroguanylin or guanylin. Specifically, a leaf and a stem are suitably used as the aerial part, and only a leaf or a stem may be used, or both a leaf and a stem may be used.

In the production method of the present invention, the plant of the genus Eleutherococcus used can be any of a raw plant as it is, a dried product, a dried powder (prepared, for example, by freeze-drying a plant of the genus Eleutherococcus and powdering the freeze-dried plant in a mortar or the like), a juice, and the like. In the present specification, the hot water extract of the aerial part of the plant of the genus Eleutherococcus may be in any form such as a liquid or a solid such as a powder.

In the production method of the present invention, the hot water extract of the aerial part of the plant of the genus Eleutherococcus is obtained, for example, by adding water having a mass 1 to 30 times a mass of the aerial part of the plant of the genus Eleutherococcus to the aerial part of the plant of the genus Eleutherococcus, adding a predetermined amount of sodium bicarbonate, and then performing hot water extraction at a high temperature (for example, extraction with hot water at 110 to 130°C for 30 to 60 minutes). Thereafter, solids may be removed, in which case a liquid obtained by separating the extract into the liquid and a residue can be used as the hot water extract of the aerial part of the plant of the genus Eleutherococcus. The separation may be performed, for example, by suction filtration of the extract, in which case the obtained filtrate may be used as the hot water extract of the aerial part of the plant of the genus Eleutherococcus. In addition, for example, the obtained filtrate further heated may be used liquid as the hot water extract of the aerial part of the plant of the genus Eleutherococcus. Thereafter, for example, the liquid may be concentrated 2 to 15 times, added with an excipient, and sterilized (for example, 110 to 130°C for 15 to 60 minutes) to obtain the hot water extract of the aerial part of the plant of the genus Eleutherococcus. Examples of the excipient include dextrin, starch, maltose, and sorbitol. Furthermore, then, for example, a drying treatment such as spray drying or freeze drying may be followed by a sieving treatment, and the obtained powder may be used as the hot water extract of the aerial part of the plant of the genus Eleutherococcus. As regards a timing for adding sodium bicarbonate, sodium bicarbonate may be first added together with the raw material as described above, or may be added when the liquid obtained by separation is further heat-treated. From the viewpoint of the effect of promoting expression of uroguanylin or guanylin, sodium bicarbonate is preferably first added together with the raw material (before hot water extraction). As regards a concentration of sodium bicarbonate to be added, sodium bicarbonate is added, for example, at a concentration of 0.05 to 10 mass%, 0.1 to 5.0 mass%, 0.1 to 3.0 mass%, 0.2 to 2.0 mass%, 0.3 to 1.5 mass%, or 0.5 to 1.0 mass% with respect to a total mass of water as an extraction solvent.

An example of the method for producing a hot water extract of an aerial part of a plant of the genus Eleutherococcus according to the present invention will be described. A method comprising the steps of: adding water, having a mass 2 to 20 times a mass of a dried product of an aerial part (leaves and stems) of a plant of the genus Eleutherococcus and containing a predetermined amount of sodium bicarbonate, to the dried product; performing extraction with hot water at 110 to 130°C for 30 to 60 minutes; collecting a supernatant liquid by centrifugation; suction-filtering the collected supernatant liquid; and obtaining a freeze-dried powder of the obtained filtrate as the hot water extract of the aerial part of the plant of the genus Eleutherococcus.

Since the hot water extract of the aerial part of the plant of the genus Eleutherococcus obtained by the production method of the present invention is excellent in effect of promoting expression of uroguanylin or guanylin, an excellent effect of promoting intestinal peristalsis, an excellent effect of suppressing intestinal inflammation, and an excellent effect of improving intestinal barrier function can be obtained. Since the functions and conditions of the intestines can thus be improved, it is suitably used for the treatment of constipation (including chronic constipation) and constipation type irritable bowel syndrome, and the like. The hot water extract of the aerial part of the plant of the genus Eleutherococcus can promote, for example, expression of uroguanylin or guanylin in a subject having normal functions and conditions of the intestines, and can also promote expression of uroguanylin or guanylin in a subject having poor functions and conditions of the intestines, more specifically, a subject in a condition of reduced intestinal peristalsis, a subject in a condition in which intestinal inflammation has occurred, or a subject in a condition of a reduced intestinal barrier function. The hot water extract of the aerial part of the plant of the genus Eleutherococcus is administered to mammals such as rodents including mice, rats, hamsters, and guinea pigs, primates including humans, chimpanzees, and rhesus monkeys, livestock including pigs, cows, goats, horses, and sheep, and pets including dogs and cats. A preferred subject is a human.

### (2. Composition for promoting expression of uroguanylin or guanylin)

A composition for promoting expression of uroguanylin or guanylin of the present invention contains as an active ingredient a hot water extract of an aerial part of a plant of the genus Eleutherococcus.

The hot water extract of the aerial part of the plant of the genus Eleutherococcus contained in the composition for promoting expression of uroguanylin or guanylin of the present invention is produced, for example, by adding sodium bicarbonate. The details of the production method are the same as described above.

The plant of the genus Eleutherococcus to be used is the same as described above, and is preferably Eleutherococcus senticosus.

The composition for promoting expression of uroguanylin or guanylin of the present invention can enhance expression of uroguanylin or guanylin to stimulate the guanylate cyclase C receptor present in intestinal mucosal epithelial cells and to increase the intracellular cGMP concentration, and thus can provide an excellent effect of promoting intestinal peristalsis, an excellent effect of suppressing intestinal inflammation, and an excellent effect of improving intestinal barrier function and improve the functions and conditions of the intestines. Therefore, it is suitably used for the treatment of constipation (including chronic constipation) and constipation type irritable bowel syndrome, and the like. Details of the subject to which the composition is to be applied are the same as described above.

### (3. Composition for promoting intestinal peristalsis, composition for suppressing intestinal inflammation, or composition for improving intestinal barrier function)

A composition for promoting intestinal peristalsis, a composition for suppressing intestinal inflammation, or a composition for improving intestinal barrier function of the present invention contains as an active ingredient a hot water extract of an aerial part of a plant of the genus Eleutherococcus produced by adding sodium bicarbonate.

The method for producing the hot water extract of the aerial part of the plant of the genus Eleutherococcus to which sodium bicarbonate is to be added is the same as described above.

The plant of the genus Eleutherococcus to be used is the same as described above, and is preferably Eleutherococcus senticosus.

The composition for promoting intestinal peristalsis, the composition for suppressing intestinal inflammation or the composition for improving intestinal barrier function of the present invention contains as an active ingredient the hot water extract of the aerial part of the plant of the genus Eleutherococcus excellent in effect of enhancing expression of uroguanylin or guanylin, and thus can enhance expression of uroguanylin or guanylin to stimulate the guanylate cyclase C receptor present in intestinal mucosal epithelial cells and to increase the intracellular cGMP concentration. An increase in the concentration of cGMP within the cell results in the activation of the cystic fibrosis transmembrane regulator (CFTR), which is present in the apical membrane of intestinal epithelial cells. This leads to the secretion of chloride ions and other substances into the intestinal lumen, accompanied by an increase in osmotic pressure within the intestinal lumen. This, in turn, facilitates the movement of water into the intestinal lumen and promotes intestinal peristalsis. In addition, an increase in intracellular cGMP concentration suppresses intestinal inflammation, thereby suppressing production of an inflammatory marker calprotectin, and production of an inflammatory cytokine TNF-α. In addition, an increase in intracellular cGMP concentration promotes expression of claudin, junctional adhesion molecule A (JAM-A), occludin, and the like constituting a tight junction that mechanically connects intestinal epithelial cells of the intestinal tract, thereby making it possible to improve the intestinal barrier function, and to prevent invasion of foreign substances such as intestinal bacteria, pathogenic bacteria, and toxins. The composition for promoting intestinal peristalsis, the composition for suppressing intestinal inflammation, or the composition for improving intestinal barrier function of the present invention is suitably used, for example, for the treatment of constipation (including chronic constipation) and constipation type irritable bowel syndrome, and the like. For example, the effect of promoting intestinal peristalsis, the effect of suppressing intestinal inflammation, and the effect of improving intestinal barrier function may be obtained by enhancing expression of uroguanylin or guanylin to stimulate the guanylate cyclase C receptor present in intestinal mucosal epithelial cells and to increase the intracellular cGMP concentration. Details of the subject to which the composition is to be applied are the same as described above.

Each of the compositions according to the present invention described above can be processed into a form suitable for foods and beverages, such as a granular form, a particulate form, a tablet form, a capsule form, a gel form, a cream form, a paste form, a suspension form, an aqueous solution form, an emulsion form, or a powder form, by a conventional method. In addition, an excipient, a binder, a lubricant, a colorant, a disintegrant, a thickener, a preservative, a stabilizer, a pH adjuster, or the like that is usually used can be added. In addition, the composition can be applied to supplements, foods and beverages, functional foods, foods for sick persons, foods for specified health uses, and the like, which are based on the concept of intestinal function improvement and indicate to that effect as necessary, and can also be used in feed for mammals and the like, pet foods, supplements for pets, and the like. An administration method may be appropriately selected as long as the method provides the effects of the present invention, including oral administration, intravenous administration, intraperitoneal administration, intradermal administration, and sublingual administration.

### (4. Conclusion)

As described above, the method for producing a hot water extract of an aerial part of a plant of the genus Eleutherococcus according to the present invention can efficiently produce a hot water extract of an aerial part of a plant of the genus Eleutherococcus excellent in effect of promoting expression of uroguanylin or guanylin, effect of promoting intestinal peristalsis, effect of suppressing intestinal inflammation, and effect of improving intestinal barrier function by adding sodium bicarbonate. A hot water extract of an aerial part of the plant of the genus Eleutherococcus produced by the method according to the present invention can effectively promote expression of uroguanylin or guanylin, stimulate a guanylate cyclase C receptor present in intestinal mucosal epithelial cells and then increase an intracellular cGMP concentration. As a result, the effect of promoting intestinal peristalsis, an effect of suppressing intestinal inflammation, and an effect of improving intestinal barrier function can be obtained, thereby improving the functions and conditions of the intestines.

In addition, the composition for promoting expression of uroguanylin or guanylin according to the present invention contains as an active ingredient a hot water extract of an aerial part of a plant of the genus Eleutherococcus, stimulates a guanylate cyclase C receptor present in intestinal mucosal epithelial cells and thus can increase the intracellular cGMP concentration. This can result in obtainment of an excellent effect of promoting intestinal peristalsis, an excellent effect of suppressing intestinal inflammation, and an excellent effect of improving intestinal barrier function, and improvement in functions and conditions of the intestines.

Further, the composition for promoting intestinal peristalsis, the composition for suppressing intestinal inflammation and the composition for improving intestinal barrier function according to the present invention contain as an active ingredient the hot water extract of the aerial part of the plant of the genus Eleutherococcus produced by adding sodium bicarbonate, and thus can enhance expression of uroguanylin or guanylin, stimulate the guanylate cyclase C receptor present in intestinal mucosal epithelial cells and then increase the intracellular cGMP concentration. For example, they are suitably used for the treatment of constipation (including chronic constipation) and constipation type irritable bowel syndrome.

### EXAMPLES

Hereinafter, the present invention will be specifically described with reference to Examples. However, the present invention is not limited to these Examples.

### (Example 1)

The influence of a hot water extract of an aerial part of Eleutherococcus senticosus (hereinafter referred to as "hot water extract of Eleutherococcus senticosus") on a relative expression level of guanylin was verified.

### (Production of hot water extract of Eleutherococcus senticosus)

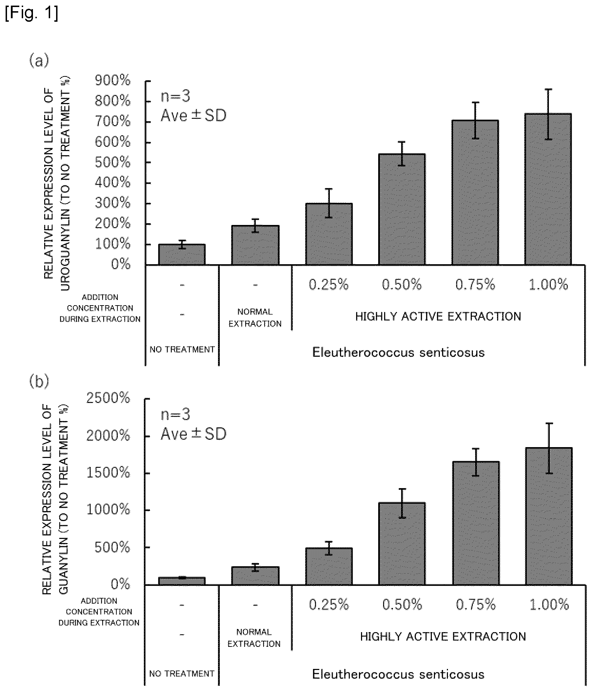

Water (containing 0.25 mass%, 0.50 mass%, 0.75 mass%, and 1.00 mass% of sodium bicarbonate with respect to the total mass of water) having a mass 15 times the mass of a dried product of the aerial part of Eleutherococcus senticosus was added to the dried product, and extraction was performed at 121°C for 45 minutes. After the extraction, centrifugation was performed at 5,000 × g for 10 minutes, and a supernatant liquid was collected. The collected supernatant liquid was subjected to suction filtration, and a freeze-dried powder of the obtained filtrate was defined as a hot water extract of Eleutherococcus senticosus ("highly active extraction" in Fig. 1) (Example). On the other hand, a freeze-dried powder obtained by adding water (not containing sodium bicarbonate) having a mass 15 times the mass of the dried product of the aerial part of Eleutherococcus senticosus to the dried product, and performing extraction and preparation in the same manner as described above is referred to as "normal extraction" (Comparative Example) in Fig. 1.

### (Test method)

A human colon cancer-derived cell line (T84, provider: American Type Culture Collection) was suspended in a DMEM: F-12 medium [adjusted by using product names: Dulbecco's modified Eagle's medium "NISSUI" 2 powder and Ham's F12 medium "NISSUI" (manufactured by NISSUI PHARMACEUTICAL CO., LTD.) each in an amount half the specified amount] supplemented with 10% fetal bovine serum (FBS), seeded at a density of 18000 cells/well in a 96 well microplate, and cultured at 37°C under 5% CO2 for 24 hours. The medium in each well was replaced with 135 µL of a DMEM: F-12 medium not containing FBS, and 15 µL of the evaluation sample solution was added to each well. The evaluation sample solutions were prepared by dissolving in the evaluation sample, such as the hot water extract of Eleutherococcus senticosus, in DMEM: F-12 medium not containing FBS to achieve a concentration of 5000 µg/mL, which is 10 times the target final concentration of 500 µg/mL. In addition, only 15 µL of the DMEM: F-12 medium not containing FBS was added to a no treatment group as a comparative control. After the cells were cultured at 37°C under 5% CO2 for 4 hours, RNA extraction was performed on the cells using TRIzol reagent (manufactured by Life Technologies Corporation), cDNA synthesis was performed based on the RNA using ReverTra Ace qPCR RT Master Mix with gDNA Remover (manufactured by Toyobo Life Science Inc.), and the reaction product was diluted 10 times with nuclease-free water to obtain a template for real-time PCR.

In the PCR reaction, SEQ ID NOs: 1 and 2 were used as primers for detecting the uroguanylin gene, and SEQ ID NOs: 3 and 4 were used as primers for detecting the guanylin gene. The glyceraldehyde-3 phosphate dehydrogenase (GAPDH) gene was used as an internal standard gene for correcting expression of a target gene, and SEQ ID NOs: 5 and 6 were used as primers for detection thereof. The real-time PCR reaction was performed using a real-time PCR reaction kit (product name: PowerUP SYBR Green Master mix, manufactured by Life Technologies Corporation) and a real-time PCR analysis system (product name: QuantStudio3, manufactured by Life Technologies Corporation). Ten (10) µL of the PCR reaction solution was incubated at 50°C for 2 minutes and at 95°C for 2 minutes (initially denatured), followed by 40 cycles of denaturation at 95°C for 15 seconds and annealing and elongation at 68°C for 60 seconds.
SEQ ID NO: 1: 5'-GCTGCAGAGCACACAGTCAG-3'
SEQ ID NO: 2: 5'-TGTGCCTCCAGGTCACTCAG-3'
SEQ ID NO: 3: 5'-GATACTCCACTCCCAGCAGC-3'
SEQ ID NO: 4: 5'-TTGAAGTGGCAGGGAAGGAC-3'
SEQ ID NO: 5: 5'-GAAGGTCGGAGTCAACGGATT-3'
SEQ ID NO: 6: 5'-CGCTCCTGGAAGATGGTGAT-3'

Each sequence was designed using Primer-BLAST published by NCBI.

The Cq value obtained by real-time PCR was used to calculate a relative expression level of the target gene (to no treatment group %) by the ΔΔCt method.

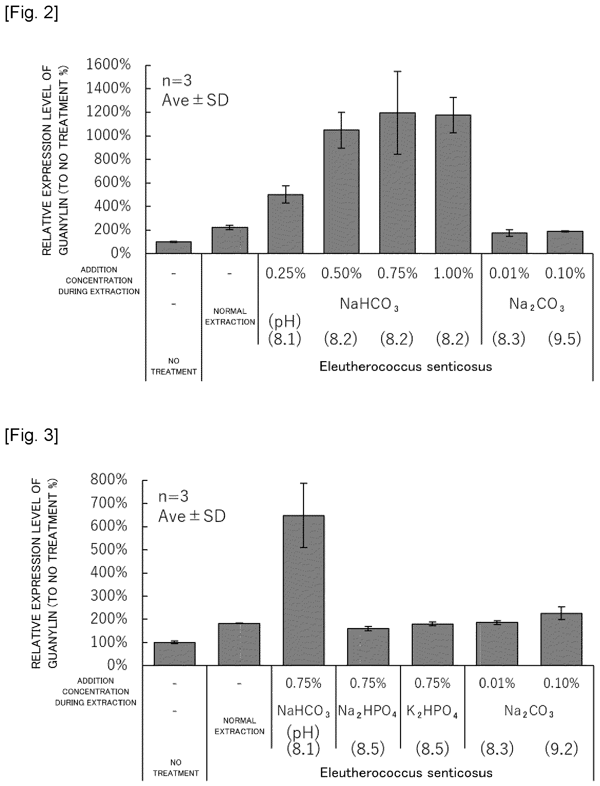

The results are shown in Fig. 1. In Fig. 1, the "addition concentration (%) during extraction" represents the sodium bicarbonate concentration of the aqueous solution used in producing the hot water extract of Eleutherococcus senticosus (hereinafter, the same applies to Figs. 2 and 3). In a normal extraction sample treatment group, the relative expression level of uroguanylin (Fig. 1(a)) and the relative expression level of guanylin (Fig. 1(b)) were slightly increased as compared with that in the no treatment group. However, in a group in which the sample extracted by adding sodium bicarbonate was treated, the relative expression level of uroguanylin (Fig. 1(a)) and the relative expression level of guanylin (Fig. 1(b)) were greatly increased in a manner of being dependent on the concentration of sodium bicarbonate. From this fact, it was shown that the hot water extract of Eleutherococcus senticosus of the Example produced by adding sodium bicarbonate promoted expression of uroguanylin and guanylin.

### (Example 2)

The influence of the method for producing a hot water extract of Eleutherococcus senticosus on the relative expression level of guanylin was verified.

### (Addition of other alkali sources)

In Example 1, it was considered that the increase in relative expression level of guanylin when using the hot water extract of Eleutherococcus senticosus produced by adding sodium bicarbonate was due to the alkaline condition employed during extraction. Therefore, alkali sources other than sodium bicarbonate were used to perform the following comparative examination.

The relative expression level of guanylin was measured using the hot water extract of Eleutherococcus senticosus produced by adding sodium bicarbonate similar to that in Example 1 ("NaHCO3" in Fig. 2) (Example) and a hot water extract of Eleutherococcus senticosus produced using sodium carbonate instead of sodium bicarbonate in Example 1 ("Na2CO3" in Fig. 2) (Comparative Example). The measurement of the relative expression level of guanylin was performed in the same manner as in Example 1. In FIG. 2, the "normal extraction" (Comparative Example) is the same as that in FIG. 1.

The results are shown in Fig. 2. In Fig. 2, the value of pH represents a pH of the aqueous solution used in producing the hot water extract of Eleutherococcus senticosus. The relative expression level of guanylin increased depending on the concentration of sodium bicarbonate when using the hot water extract of Eleutherococcus senticosus produced by adding sodium bicarbonate, whereas the relative expression level of guanylin when using the hot water extract of Eleutherococcus senticosus produced by adding sodium carbonate was comparable to that when using the normal extract, regardless of whether the concentration of sodium carbonate in the aqueous solution used in production was 0.01 mass% or 0.10 mass%. When 0.01 mass% of sodium carbonate was added, the pH of the aqueous solution used in producing the hot water extract of Eleutherococcus senticosus was 8.3, which was comparable to the pH (8.1 to 8.2) of the aqueous solution when 0.25 to 1.00 mass% of sodium bicarbonate was added.

In addition, the relative expression level of guanylin was measured in the same manner as in Example 1, using a hot water extract of Eleutherococcus senticosus produced by adding disodium hydrogen phosphate ("Na2HPO4" in Fig. 3) (Comparative Example) and a hot water extract of Eleutherococcus senticosus produced by adding dipotassium hydrogen phosphate ("K2HPO4" in Fig. 3) (Comparative Example), as other alkali sources. The concentrations of disodium hydrogen phosphate and dipotassium hydrogen phosphate added to the aqueous solution used in production were 0.75 mass%, which was the same as that of sodium bicarbonate. In FIG. 3, the "normal extraction" (Comparative Example) is the same as that in FIG. 1.

The results are shown in Fig. 3. The relative expression level of guanylin increased when using the hot water extract of Eleutherococcus senticosus produced by adding sodium bicarbonate, whereas the relative expression level of guanylin when using the hot water extract of Eleutherococcus senticosus produced by adding disodium hydrogen phosphate or dipotassium hydrogen phosphate was comparable to that when using the normal extract, although the concentration of disodium hydrogen phosphate or dipotassium hydrogen phosphate added was the same as that of sodium bicarbonate, and the aqueous solution used in production was alkaline and had a comparable pH.

From these facts, it is considered that the reason why the hot water extract of Eleutherococcus senticosus produced by adding sodium bicarbonate promotes expression of guanylin is not merely due to extraction performed under the alkaline condition but due to extraction performed using sodium bicarbonate.

### (Timing for adding sodium bicarbonate)

The influence of the timing for adding sodium bicarbonate on the relative expression level of guanylin in the production process for the hot water extract of Eleutherococcus senticosus was verified.

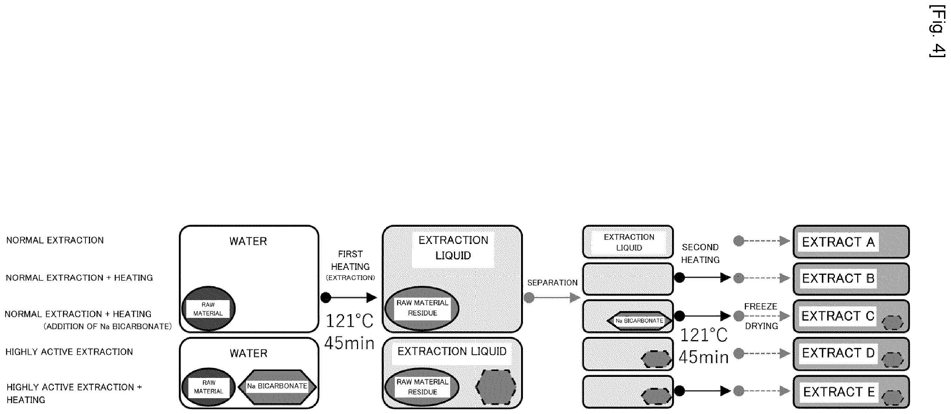

Extracts obtained by five patterns of extraction methods (Fig. 4) were compared and examined. Sodium bicarbonate was not added for extracts A to C, and sodium bicarbonate attaining 0.75 mass% as an aqueous solution was added for extracts D and E, and hot water extraction was performed by a first heat treatment at 121°C for 45 minutes. All the extracts were centrifuged and filtered to obtain extracts as supernatant liquids, and then the same amount of sodium bicarbonate as that for the extracts D and E was added to the extract C. The extracts B, C, and E were subjected to a second heat treatment at 121°C for 45 minutes. All the extracts were freeze-dried to obtain hot water extracts of Eleutherococcus senticosus. The relative expression level of guanylin was measured using the extracts A to E in the same manner as in Example 1.

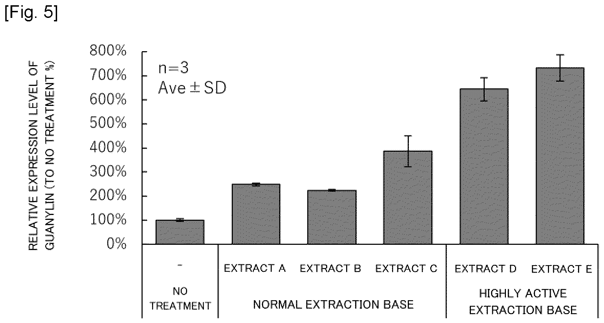

The results are shown in Fig. 5. As compared with the no treatment sample, the extract A and the extract B produced without adding sodium bicarbonate provided a relative expression level of guanylin about 2 times, but the extract C produced by adding sodium bicarbonate and subjected to the second heat treatment provided a relative expression level of guanylin increased about 4 times. On the other hand, the extract D and the extract E produced by adding sodium bicarbonate and subjected to the first heat treatment provided a relative expression level of guanylin dramatically increased to about 6.5 to 7.5 times. From this fact, it was shown that, by adding sodium bicarbonate during hot water extraction, a hot water extract of Eleutherococcus senticosus having a high effect of promoting guanylin expression can be obtained.

### (Example 3)

For extracts of various plants produced by adding sodium bicarbonate, the relative expression level of guanylin was measured.

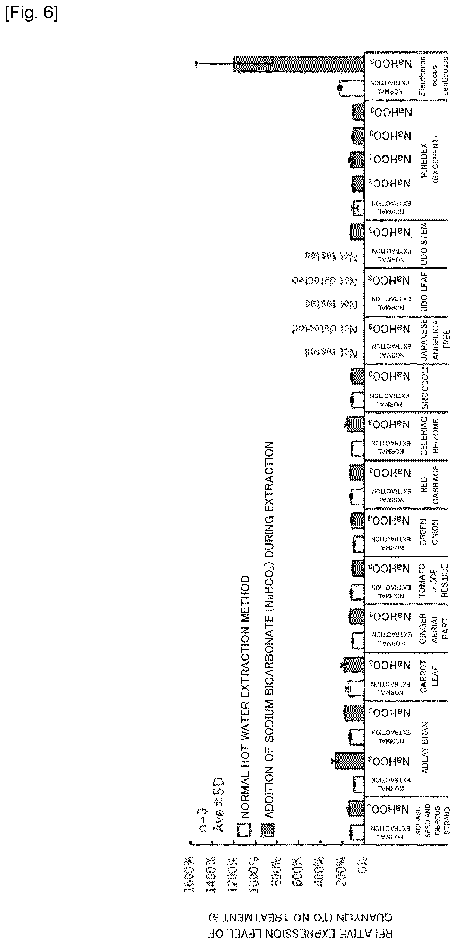

Hot water extraction was performed, in the same manner as in Example 1, on each of the squash seeds and fibrous strands, adlay bran, carrot leaves, ginger aerial parts, tomato juice residue, green onion, red cabbage, celeriac rhizome, broccoli, Japanese angelica tree, udo leaves, udo stems, Pinedex (Matsutani Chemical Industry Co., Ltd.), and aerial parts of Eleutherococcus senticosus, thereby obtaining each extract. The concentration of sodium bicarbonate added was 0.75 mass% as a concentration as an aqueous solution. For each of these extracts, the relative expression level of guanylin was measured in the same manner as in Example 1. In FIG. 6, the "normal extraction" is the same as that in FIG. 1.

The results are shown in Fig. 6. The relative expression level of guanylin did not increase when using the hot water extracts of the plants other than Eleutherococcus senticosus, whereas the relative expression level of guanylin dramatically increased when using the hot water extract of Eleutherococcus senticosus as compared with that when using the normal extraction, which showed an increase about 12 times that of the no treatment group. From this fact, it was shown that the hot water extract of Eleutherococcus senticosus obtained by adding sodium bicarbonate has an effect of specifically increasing the relative expression level of guanylin, unlike the hot water extracts of the other plants.

### (Example 4)

Mice were fed with the hot water extracts of Eleutherococcus senticosus and subjected to various functional evaluations.

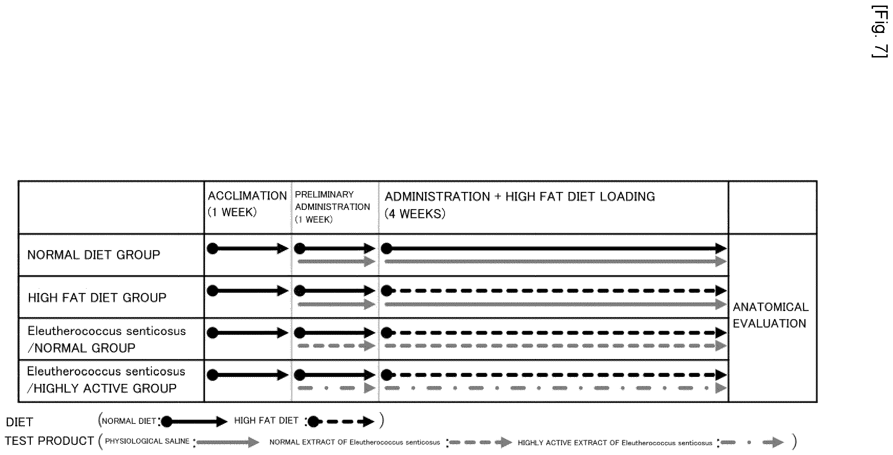

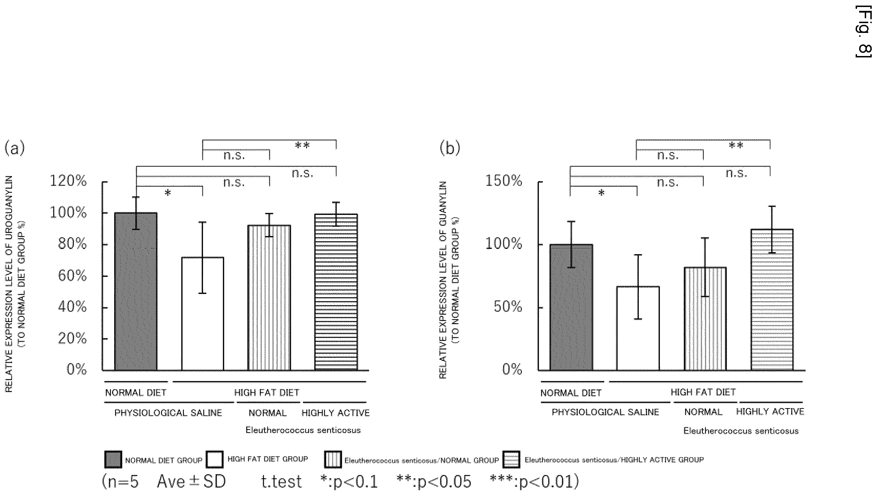

Mice (C57BL/6NCrSlc, female, 7 weeks old) were divided into the following four groups. The administration schedule for each group is shown in Fig. 7.
- Normal diet group (control group): fed with 0.2 mL of physiological saline (Otsuka Normal Saline: manufactured by Otsuka Pharmaceutical Co., Ltd.) and normal diet (CE-2: manufactured by CLEA Japan, Inc.) for 5 weeks ("normal diet" in Figs. 8 to 12)
- High fat diet group: fed with 0.2 mL of physiological saline and normal diet for 1 week, and then fed with 0.2 mL of physiological saline and high fat diet (High Fat Diet 32: manufactured by CLEA Japan, Inc.) for 4 weeks ("high fat diet" in Figs. 8 to 12)
- Eleutherococcus senticosus/normal group: fed with a normal diet for 1 week, followed by a high fat diet for 4 weeks. In addition, they were fed with the hot water extract of Eleutherococcus senticosus extracted in the same manner as in Example 1, without addition of sodium bicarbonate, at a dose of 500 mg/kg per day for these 5 weeks ("Eleutherococcus senticosus/normal" in Figs. 8 to 12).
- Eleutherococcus senticosus/highly active group: fed with a normal diet for 1 week, followed by a high fat diet for 4 weeks. In addition, they were fed with the hot water extract of Eleutherococcus senticosus extracted in the same manner as in Example 1, with addition of 0.75 mass% of sodium bicarbonate as an aqueous solution, at a dose of 500 mg/kg per day for these 5 weeks ("Eleutherococcus senticosus/highly active" in Figs. 8 to 12).

Feeding with the hot water extract of Eleutherococcus senticosus was performed by oral gavage using an oral sonde, and mice were administered with a predetermined amount of the hot water extract of Eleutherococcus senticosus dissolved in 0.2 mL of physiological saline (Otsuka Normal Saline: manufactured by Otsuka Pharmaceutical Co., Ltd.). In addition, in the normal diet group and the high fat diet group, 0.2 mL of physiological saline (the same as described above) not containing a hot water extract of Eleutherococcus senticosus was subjected to oral gavage using an oral sonde.

For each group, mice were dissected after feeding for a total of 5 weeks, and the following evaluation was performed (Fig. 7).

### (Evaluation of uroguanylin expression level and guanylin expression level)

Several papers such as Waldman, S.A., & Camilleri, M. (2018). Guanylate cyclase-C as a therapeutic target in gastrointestinal disorders. Gut, 67 (8), 1543-1552. and Sindic, A.(2013). Current Understanding of Guanylin Peptides Actions. ISRN Nephrology, 2013, 1-17 have reported that the expression level of uroguanylin is high in the upper part of the small intestine (jejunum) and the expression level of guanylin is high in the large intestine. Therefore, the uroguanylin expression level and the guanylin expression level in these regions were evaluated.

In the small intestine obtained by dissection, a region of 2 cm on the stomach side was excluded as the duodenum, a 1/3 region from the stomach side of the remaining small intestine was defined as the jejunum, and a 2/3 region on the cecum side was defined as the ileum. A central 5 mm region of each of the jejunum, the ileum, and the large intestine was collected as tissue thereof, RNA extraction was performed on the tissue using TRIzol Reagent (manufactured by Life Technologies Corporation), cDNA synthesis was performed based on the RNA using ReverTra Ace qPCR RT Master Mix with gDNA Remover (manufactured by Toyobo Life Science Inc.), and the reaction product was diluted 10 times with nuclease-free water to obtain a template for real-time PCR.

In the PCR reaction, SEQ ID NOs: 7 and 8 were used as primers for detecting the uroguanylin gene of mice, and SEQ ID NOs: 9 and 10 were used as primers for detecting the guanylin gene of mice. The mouse GAPDH gene was used as an internal standard gene for correcting expression of the target gene, and SEQ ID NOs: 11 and 12 were used as primers thereof. The real-time PCR reaction was performed using a real-time PCR reaction kit (product name: PowerUP SYBR Green Master mix, manufactured by Life Technologies Corporation) and a real-time PCR analysis system (product name: QuantStudio3, manufactured by Life Technologies Corporation). Ten (10) µL of the PCR reaction solution was incubated at 50°C for 2 minutes and at 95°C for 2 minutes (initially denatured), followed by 40 cycles of denaturation at 95°C for 15 seconds and annealing and elongation at 61°C for 60 seconds.
SEQ ID NO: 7: 5'-AGGAGATGTCCAATCCCCAG-3'
SEQ ID NO: 8: 5'-ACAGTTCACATTCGTCGGTGG-3'
SEQ ID NO: 9: 5'-GATCCTGCAGAGGCTAGAGG-3'
SEQ ID NO: 10: 5'-AAGGCAAGCGATGTCACTCT-3'
SEQ ID NO: 11: 5'-ACCCAGAAGACTGTGGATGG-3'
SEQ ID NO: 12: 5'-TCAGCTCTGGGATGACCTTG-3'

The primers of SEQ ID NOs: 7 to 10 were constructed with reference to Ikpa, P.T., Sleddens, H.F.B.M., Steinbrecher, K.A., Peppelenbosch, M.P., de Jonge, H.R., Smits, R., & Bijvelds, M.J.C. (2016). Guanylin and uroguanylin are produced by mouse intestinal epithelial cells of columnar and secretory lineage. Histochemistry and Cell Biology, 146 (4), 445-455., and the primers of SEQ ID NOs: 11 and 12 were designed using Primer-BLAST published by NCBI.

The Cq value obtained by real-time PCR was used to calculate a relative expression level of the target gene (to normal diet group %) by the ΔΔCt method.

The results are shown in Fig. 8. Both the uroguanylin expression level in the jejunum (Fig. 8(a)) and the guanylin expression level in the large intestine (Fig. 8(b)) decreased in the high fat diet group as compared with those in the normal diet group (control group), while the decreases in these expression levels were suppressed in the Eleutherococcus senticosus/normal group. Further, these expression levels significantly increased in the Eleutherococcus senticosus/highly active group as compared with those in the high fat diet group. From these facts, it was shown that the hot water extract of Eleutherococcus senticosus of the Example increases the expression levels of uroguanylin and guanylin decreased by the high fat diet.

### (Evaluation of production level of cGMP)

Since it had been found that the uroguanylin and guanylin expression levels in the intestinal tissue were increased by the hot water extract of Eleutherococcus senticosus of the Example, it was expected that the amount of cGMP in the tissue was increased by the action thereof. Therefore, the amount of cGMP was measured according to the attached manual using the Cyclic cGMP ELISA Kit (manufactured by Cayman Chemical) using a central 1/3 region of the small intestine (corresponding to the first half of the ileum) and the large intestine (a region not used in the measurement of RNA described above or TNF-α which will be described later) as samples. The cGMP measured value was corrected by measuring the concentration of protein extracted from the tested tissue using TaKaRa BCA Protein Assay Kit (manufactured by Takara Bio Inc.).

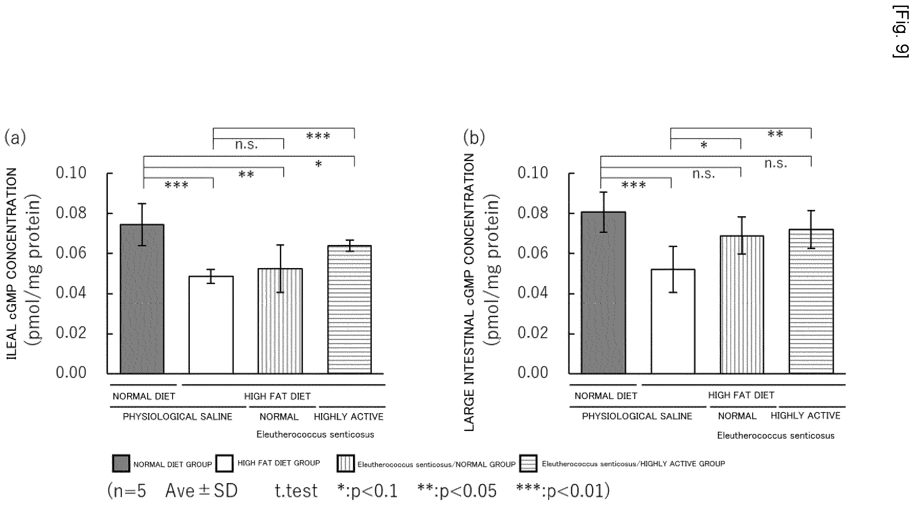

The results are shown in Fig. 9. For both the ileum sample (Fig. 9(a)) and the large intestine sample (Fig. 9(b)), the cGMP amount was decreased in the high fat diet group as compared with that of the normal diet group (control group), while the decrease in cGMP amount was suppressed in the Eleutherococcus senticosus/normal group and the Eleutherococcus senticosus/highly active group, and further, the cGMP amount in the ileum and the large intestine was significantly increased in the Eleutherococcus senticosus/highly active group as compared with that in the high fat diet group. From these facts, it was shown that the hot water extract of Eleutherococcus senticosus of the Example increases the cGMP amount decreased by the high fat diet. That is, it was revealed that the hot water extract of Eleutherococcus senticosus of the Example not only increases the expression levels of mRNA of uroguanylin and guanylin, but also increases the protein expression levels of uroguanylin and guanylin, which are functional bodies.

### (Evaluation of index related to intestinal inflammation)

The expression level of an inflammatory marker calprotectin in the large intestine and the amount of TNF-α per weight of large intestinal tissue were measured as follows.

The expression level of calprotectin (S100A8) was evaluated in the same manner as in the evaluation of the uroguanylin and guanylin expression levels described above, using SEQ ID NOs: 13 and 14 as primers for detection.
SEQ ID NO: 13: 5'-TGAGTGTCCTCAGTTTGTGCAG-3'
SEQ ID NO: 14: 5'-TGTGAGATGCCACACCCACTTT-3'
SEQ ID NOs: 13 and 14 were designed using Primer-BLAST published by NCBI.

In order to measure TNF-α derived from the large intestinal tissue, a 1 cm intestinal tract on the rectum side from the central part of the large intestine used in RNA extraction was cut out, cut in the longitudinal direction so that the lumen side was exposed, and then washed twice with penicillin-streptomycin-containing PBS. Thereafter, the tissues were cultured in a serum-free RPMI1640 medium (product name: RPMI1640 medium "NISSUI" 2 powder, manufactured by NISSUI PHARMACEUTICAL CO., LTD.) at 37°C and under 5% CO2 for 24 hours to collect a culture supernatant. The amount of TNF-α in the collected culture supernatant was measured according to the attached manual using Quantikine TNF-α ELISA Kit (manufactured by R & D systems). The TNF-α measured value was corrected by the weight of the tested tissue.

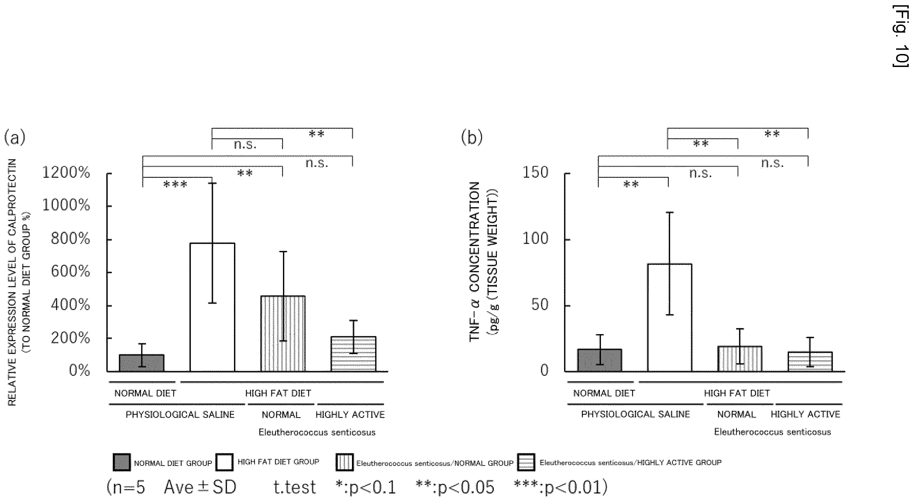

The results are shown in Fig. 10. Both the expression level of calprotectin as an inflammation marker (Fig. 10(a)) and the TNF-α amount per weight of large intestinal tissue (Fig. 10(b)) increased in the high fat diet group as compared with the normal diet group (control group). On the other hand, the calprotectin expression level decreased in the Eleutherococcus senticosus/normal group as compared with that in the high fat diet group, and further significantly decreased in the Eleutherococcus senticosus/highly active group as compared with that in the high fat diet group (Fig. 10(a)). In addition, the TNF-α amount per weight of large intestinal tissue significantly decreased in the Eleutherococcus senticosus/normal group and the Eleutherococcus senticosus/highly active group as compared with that in the high fat diet group (Fig. 10(b)). From these facts, it was shown that the hot water extract of Eleutherococcus senticosus of the Example suppresses the intestinal inflammation increased by the high fat diet.

### (Evaluation of expression level of tight junction-related gene involved in intestinal barrier function)

The expression levels of Claudin 4, JAM-A (Junctional Adhesion Molecule), and occludin, which are tight junction-related genes, in the large intestine were measured as follows.

The expression levels were evaluated, in the same manner as in the evaluation of the expression levels of uroguanylin and guanylin described above, using primers of SEQ ID NOs: 15 and 16 for the expression level of Claudin 4, primers of SEQ ID NOs: 17 and 18 for the expression level of JAM-A, and primers of SEQ ID NOs: 19 and 20 for the expression level of occludin.
SEQ ID NO: 15: 5'-CGCTACTCTTGCCATTACG-3'
SEQ ID NO: 16: 5'-ACTCAGCACACCATGACTTG-3'
SEQ ID NO: 17: 5'-ACCCTCCCTCCTTTCCTTAC-3'
SEQ ID NO: 18: 5'-CTAGGACTCTTGCCCAATCC-3'
SEQ ID NO: 19: 5'-TTGACAGTCCAATGGCCTAC-3'
SEQ ID NO: 20: 5'-CATCCACACTCAAGGTCAGAG-3'
SEQ ID NOs: 15 to 18 were constructed with reference to Gong, J., Xu, J., Zhu, W., Gao, X., Li, N, & Li, J.(2010). Epithelial-specific blockade of MyD88-dependent pathway causes spontaneous small intestinal inflammation. Clinical Immunology, 136 (2), 245-256., and SEQ ID NOs: 19 and 20 were designed using Primer-BLAST published by NCBI.

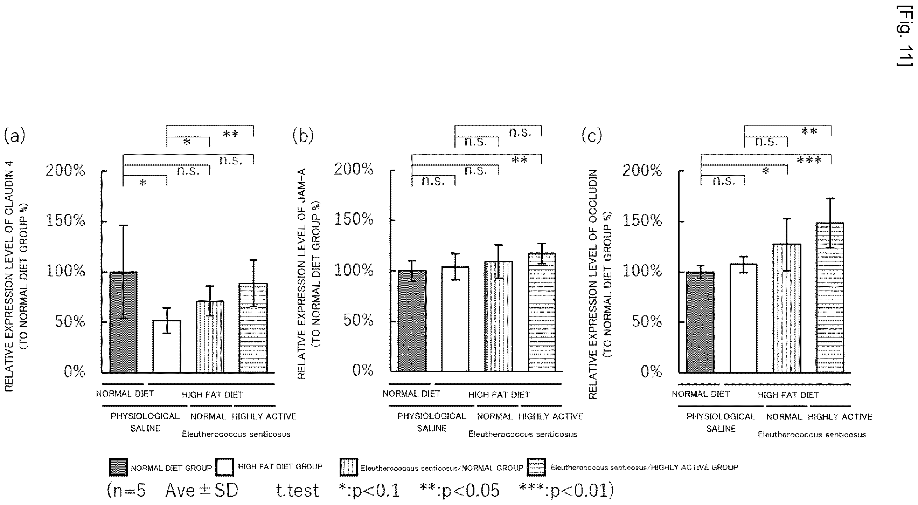

The results are shown in Fig. 11. The expression level of Claudin 4 (Fig. 11(a)), the expression level of JAM-A (Fig. 11(b)), and the expression level of occludin (Fig. 11 (c)) involved in the intestinal barrier function tended to be higher in the Eleutherococcus senticosus/highly active group. Especially, the expression levels of Claudin 4 and occludin significantly increased as compared with those in the high fat diet group (Figs. 11(a) and (c)). From these results, it was shown that the hot water extract of Eleutherococcus senticosus of the Example increases the expression levels of the tight junction-related genes of the intestines and improves the intestinal barrier function.

### (Evaluation of feces amount)

The feces amount was evaluated as follows. A total amount of feces in the final week of the test in a housing box in which five mice in each group were bred was collected using a sieve or the like so as not to contain a housing bed mat or diet residues, and then freeze-dried to measure the dry feces weight.

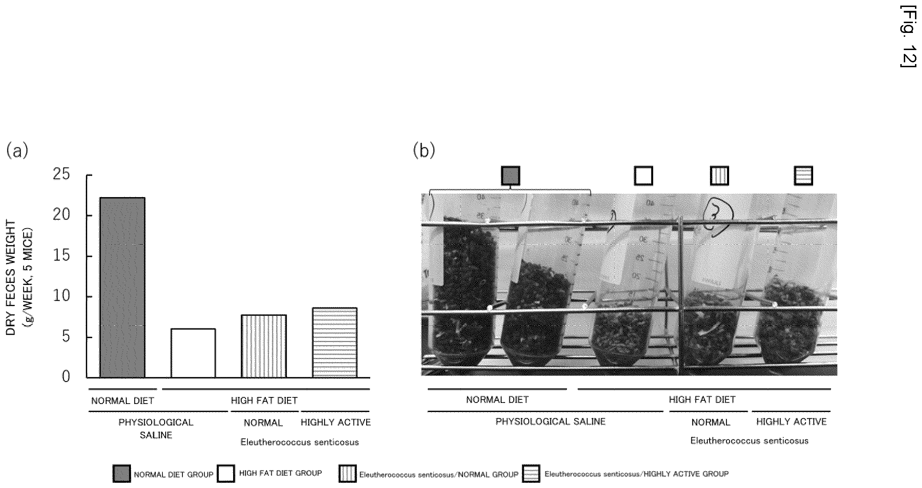

The results are shown in Fig. 12. The feces amount in the final week of the test was 27% in the high fat diet group, 35% in the Eleutherococcus senticosus/normal group, and 39% in the Eleutherococcus senticosus/highly active group, as compared with that in the normal diet group (control group), and the feces amount tended to increase in the Eleutherococcus senticosus/normal group and the Eleutherococcus senticosus/highly active group (Fig. 12(a)). From this fact, it was shown that the hot water extract of Eleutherococcus senticosus of the Example alleviates constipation-like symptoms induced by the high fat diet.

Note that various embodiments and modifications can be made without departing from the spirit and scope of the present invention in a broad sense. In addition, the above-described embodiments are for illustrating the present invention, and do not limit the scope of the present invention. That is, the scope of the present invention is defined not by the embodiments but by the claims. Various modifications made within the scope of the claims and the significance of the invention equivalent thereto are regarded as being within the scope of the present invention.

The present invention is based on Japanese Patent Application No. 2022-199475 filed on December 14, 2022. The entire specification, claims, and drawings of Japanese Patent Application No. 2022-199475 are incorporated herein by reference.

## Claims

1. A method for producing a hot water extract of an aerial part of a plant of the genus Eleutherococcus, comprising a step of adding sodium bicarbonate.

2. The production method according to claim 1, wherein the hot water extract of the aerial part of the plant of the genus Eleutherococcus promotes expression of uroguanylin or guanylin.

3. The production method according to claim 1 or 2, wherein the plant of the genus Eleutherococcus is Eleutherococcus senticosus.

4. A composition for promoting expression of uroguanylin or guanylin, comprising as an active ingredient a hot water extract of an aerial part of a plant of the genus Eleutherococcus.

5. The composition according to claim 4, wherein the hot water extract of the aerial part of the plant of the genus Eleutherococcus is produced by adding sodium bicarbonate.

6. The composition according to claim 4 or 5, wherein the plant of the genus Eleutherococcus is Eleutherococcus senticosus.

7. A composition for promoting intestinal peristalsis, comprising as an active ingredient a hot water extract of an aerial part of a plant of the genus Eleutherococcus produced by adding sodium bicarbonate.

8. The composition according to claim 7, wherein the plant of the genus Eleutherococcus is Eleutherococcus senticosus.

9. A composition for suppressing intestinal inflammation, comprising as an active ingredient a hot water extract of an aerial part of a plant of the genus Eleutherococcus produced by adding sodium bicarbonate.

10. The composition according to claim 9, wherein the plant of the genus Eleutherococcus is Eleutherococcus senticosus.

11. A composition for improving intestinal barrier function, comprising as an active ingredient a hot water extract of an aerial part of a plant of the genus Eleutherococcus produced by adding sodium bicarbonate.

12. The composition according to claim 11, wherein the plant of the genus Eleutherococcus is Eleutherococcus senticosus.
